(19)

Europäisches
Patentamt
European
Patent Office
Office européen
des brevets

(11) **EP 3 418 928 A1**

(12) **EUROPEAN PATENT APPLICATION**

(43) Date of publication:
26.12.2018 Bulletin 2018/52

(51) Int Cl.:
***G06F 19/00*** *(2018.01)*

(21) Application number: 18176391.3

(22) Date of filing: 07.06.2018

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: 23.06.2017   JP 2017123453

(71) Applicant: **Sumitomo Rubber Industries, Ltd.
Kobe-shi, Hyogo-ken (JP)**

(72) Inventors:
• BITO, Yasumasa
  Kobe-shi, Hyogo 651-0072 (JP)
• MINAGAWA, Yasuhisa
  Kobe-shi, Hyogo-ken, Hyogo 651-0072 (JP)

(74) Representative: **Manitz Finsterwald Patentanwälte
PartmbB
Martin-Greif-Strasse 1
80336 München (DE)**

(54) **METHOD FOR DEFINING BOND POTENTIAL OF POLYMER MATERIAL**

(57)    A method for defining bond potential of polymer material, which is the bond potential between at least two atoms constituting a molecular chain of the polymer material, by using a computer comprises steps S4 and S5. In the step S4, the computer obtains a potential curve indicating relationship between potential energy of the first molecular model in which the at least two particle models are associated with each other and a distance between the at least two particle models without making a structural relaxation calculation for the first molecular model by the computer, In the step S5, the computer defines the bond potential by using Morse-type function. The step S5 of defining the bond potential includes a calculation step for specifying fitting parameters ForceC and DLim of the Morse-type function such that the Morse-type function approximates the potential curve.

EP 3 418 928 A1

**Description**

**Technical Field**

The present invention relates to a method for defining bond potential of polymer material.

**Background Art**

In recent years, in order to develop a polymer material such as rubber, a simulation method (numerical calculation) for evaluating properties of the polymer material by using a computer has been proposed. In this type of simulation method, first, a plurality of particle models is set in which a plurality of atoms constituting a molecular chain of the polymer material are each modeled. Next, bond potential is defined between the two particle models. This defines a linear molecular chain model. The bond potential can be defined by using Morse-type function, for example.

In order to define the bond potential by using the Morse-type function, it is necessary to specify fitting parameters of the Morse-type function. The fitting parameters are specified by approximating the Morse-type function to a potential curve calculated based on the density functional theory, for example. However, there is room for further improvement in defining the bond potential with high accuracy.

Summary of the Invention

The present invention was made in view of the above, and a primary object thereof is to provide a method for defining the bond potential with high accuracy.

In one aspect of the present invention, a method for defining bond potential of polymer material, which is the bond potential between at least two atoms constituting a molecular chain of the polymer material, by using a computer comprises steps of inputting at least two particle models respectively modeling the at least two atoms into the computer, defining, in the computer, a first molecular model in which the at least two particle models are associated with each other, obtaining, by using the computer, a potential curve indicating relationship between potential energy of the first molecular model and a distance between the at least two particle models without making a structural relaxation calculation for the first molecular model by the computer, and defining, by the computer, the bond potential by using Morse-type function defined by the following formula (1):

$$E = \mathrm{DLim} \times \left[ exp\left( - \sqrt{\frac{ForceC}{DLim}} \times (R - R_M) \right) - 1 \right]^2 \cdots (1)$$

wherein E is potential energy, ForceC and DLim are fitting parameters of the Morse-type function, R is a distance between the two particle models, and RM is an equilibrium distance between the two particle models, and wherein the step of defining the bond potential includes a calculation step for specifying the fitting parameters ForceC and DLim of the Morse-type function such that the Morse-type function approximates the potential curve.

In another aspect of the invention, it is preferred that the method for defining bond potential of polymer material further comprises steps of inputting a second molecular model, in which the at least two particle models are dissociated, into the computer, and obtaining, by using the computer, bond dissociation energy between the at least two particle models by making the structural relaxation calculation for the second molecular model, wherein in the calculation step, the fitting parameter DLim of the Morse-type function is specified by using the obtained bond dissociation energy.

In another aspect of the invention, it is preferred that the step of defining the bond potential includes a step of specifying, in the potential curve, a first distance between the at least two particle models in an equilibrium state of the first molecular model and a second distance, which is larger than the first distance, between the at least two particle models at an increase change point at which increase of the potential energy becomes small, and in the calculation step, the fitting parameter ForceC of the Morse-type function is specified such that the Morse-type function approximates the potential curve in a region thereof between the first distance and the second distance.

**Brief Description of the Drawings**

Fig. 1 is a perspective view showing an example of a computer that executes a method for defining bond potential of polymer material of the present invention.

Fig. 2 is a structural formula of polyisoprene.

Fig. 3 is a conceptual diagram showing an example of a molecular chain model in which a molecular chain of a polymer material is modeled.

Fig. 4 is a graph showing an example of a potential curve of Morse-type function.

Fig. 5 is a flowchart showing an example of processing procedure of the method for defining the bond potential of polymer material in a first embodiment.

Fig. 6 is a conceptual diagram showing an example of a first molecular model.

Fig. 7 is a graph showing an example of the potential curve of the first molecular model.

Fig. 8 is a flowchart showing an example of the processing procedure of a bond potential definition step.

Fig. 9 is a flowchart showing an example of the processing procedure of the bond potential definition step according to a second embodiment of the present invention.

Fig. 10 is a flowchart showing an example of a calculation step according to the second embodiment of the present invention.

Fig. 11 is a flowchart showing an example of the processing procedure of the defining method according to a third embodiment of the present invention.

Fig. 12 is a conceptual diagram showing an example of a second molecular model.

Fig. 13 is a flowchart showing an example of the calculation step according to the third embodiment of the present invention.

Fig. 14 is a graph showing the potential curves of the Morse-type function of Examples 1 to 3 and Reference.

## Description of the Preferred Embodiment

An embodiment (first embodiment) of the present invention will now be described in conjunction with accompanying drawings.

The method for defining the bond potential of polymer material (hereinafter may be simply referred to as the "defining method") in this embodiment (first embodiment) is a method for defining the bond potential between two atoms among a plurality of atoms constituting a molecular chain of polymer material by computer.

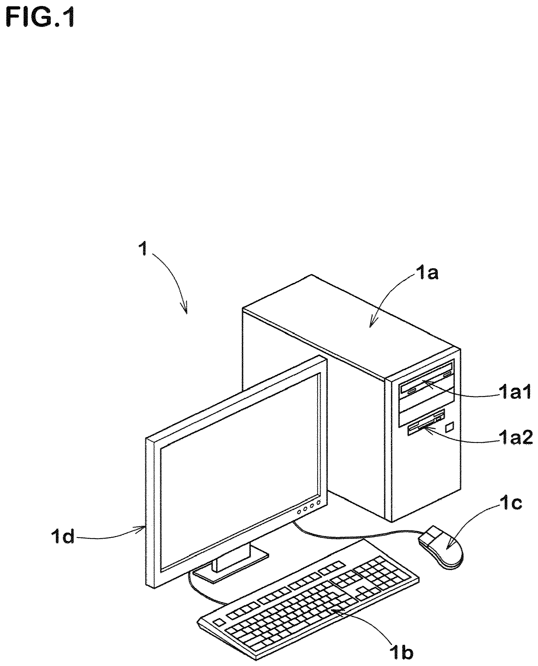

Fig. 1 is a perspective view showing an example of a computer that executes the defining method of the present invention. A computer 1 includes a main body 1a, a keyboard 1b, a mouse 1c, and a display device 1d. The main body 1a is provided with an arithmetic processing unit (CPU), a storage unit such as a ROM (Read Only Memory), a working memory, and a magnetic disk, and disk drive devices 1a1 and 1a2, for example. Further, in the storage unit, a processing procedure (program) for executing the defining method in this embodiment is stored in advance.

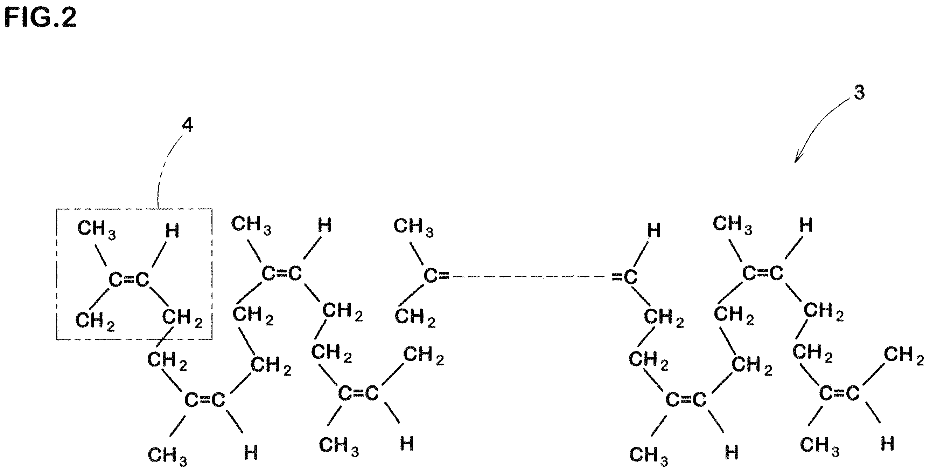

Examples of the polymer material include rubber, resin, elastomer, and the like, for example. The polymer material in this embodiment is cis-1,4 polyisoprene (hereinafter may be simply referred to as "polyisoprene"). Fig. 2 is a structural formula of polyisoprene.

A molecular chain 3 constituting polyisoprene is formed of isoprene monomers (isoprene molecules) 4 connected at a polymerization degree of n. The isoprene monomer 4 is constituted by a methine group or the like (for example, -CH=, >C=), a methylene group (-CH2-), and a methyl group (-CH3). Note that polymers other than polyisoprene may be used as the polymer material.

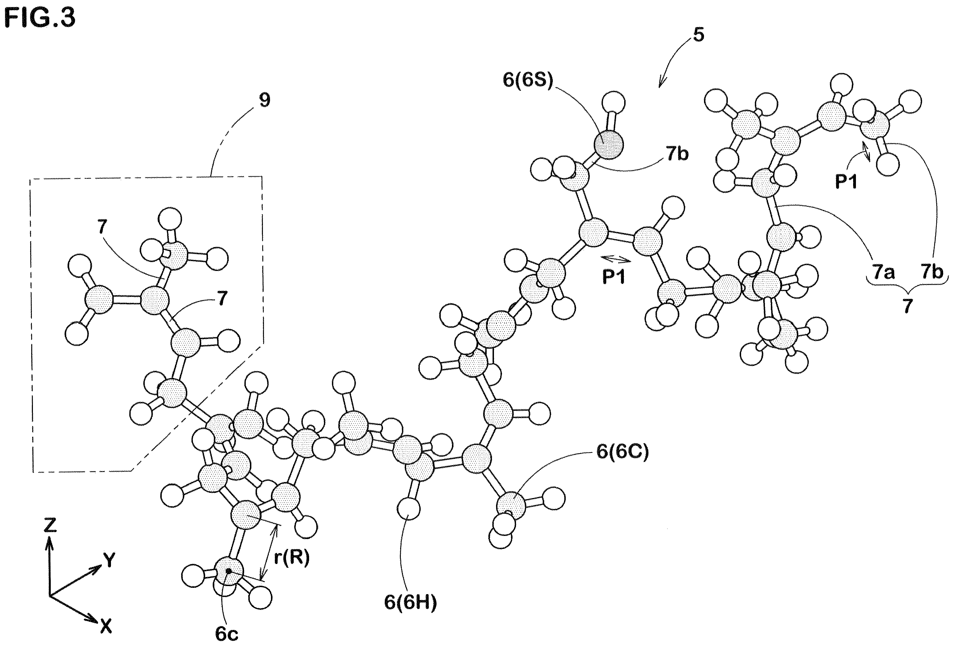

Fig. 3 is a conceptual diagram showing an example of a molecular chain model 5 in which a molecular chain of a polymer material is modeled. The molecular chain model 5 is configured as an all-atom model. The molecular chain model 5 in this embodiment is set as polyisoprene in which one sulfur atom is crosslinked. The molecular chain model 5 is configured to include a plurality of particle models 6 and bond models 7 connecting between the particle models 6.

The particle models 6 and the bond models 7 are connected based on a unit structure forming the monomer 4 of the molecular chain 3 shown in Fig. 2. Therefore, the monomer model 9 is set. This monomer model 9 is connected based on molecular weight (polymerization degree) Mn. Thereby, the molecular chain model 5 is set.

The geometry of the particle models 6 are used as initial coordinates of the atom in quantum chemical calculation performed in a later-described step S4. That is, in the particle models 6, parameters such as charge, spin, atomic coordinates, and the like are defined.

The particle models 6 in this embodiment include carbon particle models 6C modeling carbon atoms, hydrogen particle models 6H modeling hydrogen atoms, and sulfur particle models 6S modeling sulfur atoms of the molecular chain 3 shown in Fig. 2.

The bond models 7 are to bind the particle models 6. The bond models 7 in this embodiment include main chains 7a and side chains 7b. Each of the main chains 7a is to connect between the carbon particle models 6C. Each of the side chains 7b is to connect between the carbon particle model 6C and the hydrogen particle model 6H, between the carbon particle model 6C and the sulfur particle model 6S, and between the sulfur particle models 6S and the hydrogen particle model 6H. Further, the bond models 7 include single-bond bond models defining single bonds between a pair of atoms and double-bond bond models defining double bonds between a pair of atoms.

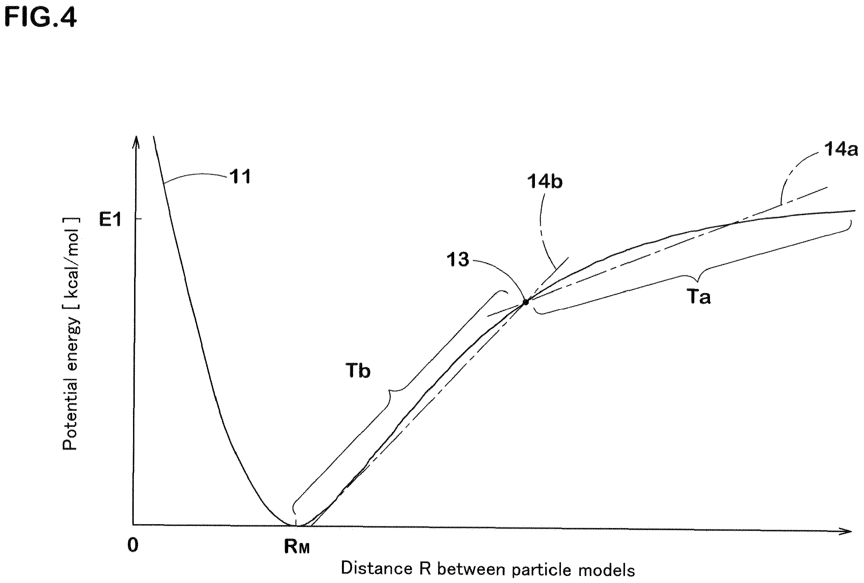

The bond model 7 is defined by bond potential P1 that causes interaction (including repulsive force and attractive force) between two adjacent particle models (i.e. atoms) 6. In the defining method in this embodiment, the bond potential P1 is defined by using Morse-type function defined by the following formula (1). Fig. 4 is a graph showing an example of a potential curve 11 of Morse-type function.

$$E = \mathrm{DLim} \times \left[ exp\left( -\sqrt{\frac{ForceC}{DLim}} \times (R - R_M) \right) - 1 \right]^2 \quad \cdots(1)$$

wherein each of the constants and variables is as follows.

E: potential energy
ForceC, DLim: fitting parameters
R: distance between two particle models
RM: equilibrium distance between two particle models

Note that the distance R and the equilibrium distance RM in the formula (1) are defined as the distances between centers 6c (shown in Fig. 3) of the particle models 6.

In the graph of Fig. 4, the smaller the distance R between two particle models 6 (shown in Fig. 3) than the equilibrium distance RM, the larger potential energy of the Morse-type function is due to the repulsive force between the particle models 6. Further, the potential energy becomes minimum when the distance R becomes the equilibrium distance RM, and no repulsive force or attractive force acts between the particle models 6. Furthermore, the potential energy becomes larger due to the attractive force between the particle models 6 when the distance R becomes larger than the equilibrium distance RM. In this way, the potential curve 11 of the Morse-type function can be used to define the repulsive force and the attractive force between the particle models 6 according to the distance R. In this specification, "potential energy" is relative energy when the total energy at the equilibrium distance RM is taken as zero.

The potential curve 11 of the Morse-type function has an increase change point 13 where the increase of the potential energy becomes smaller in a region where the distance R between the particle models 6 is larger than the equilibrium distance RM. The increase change point 13 can be specified by an intersection of a pair of straight lines 14a and 14b which approximate the potential curve 11 respectively in a region Ta where the distance R is expected to be larger than that at the increase change point 13 and in a region Tb where the distance R is expected to be smaller than that at the increase change point 13.

In the region Tb where the distance R is smaller than that at the increase change point 13, the increase of the potential energy of the Morse-type function is larger than the region Ta where the distance R is larger than that at the increase change point 13. This shows that the bond between the particle models 6 shown in Fig. 3 is strongly maintained in the region Tb where the distance R is smaller than that at the increase change point 13. Further, in the region Ta where the distance R is larger than that at the increase change point 13, cleavage or decrease of bond order occurs between the particle models 6, therefore, the increase of the potential energy is small.

The fitting parameter ForceC of the equation (1) corresponds to curvature of the potential curve 11 of the Morse-type function in the vicinity of the equilibrium distance RM. Further, the fitting parameter DLim of the equation (1) corresponds to the potential energy at boundary between the binding and dissociation (that is, bond dissociation energy E1) between the particle models 6 in the potential curve 11 of the Morse-type function. The bond dissociation energy E1 in this embodiment is defined as the potential energy at the distance R where the increase of the potential energy becomes zero in the region Ta where the distance R is larger than that at the increase change point 13. By specifying these fitting parameters ForceC and DLim, the bond potential P1 can be defined.

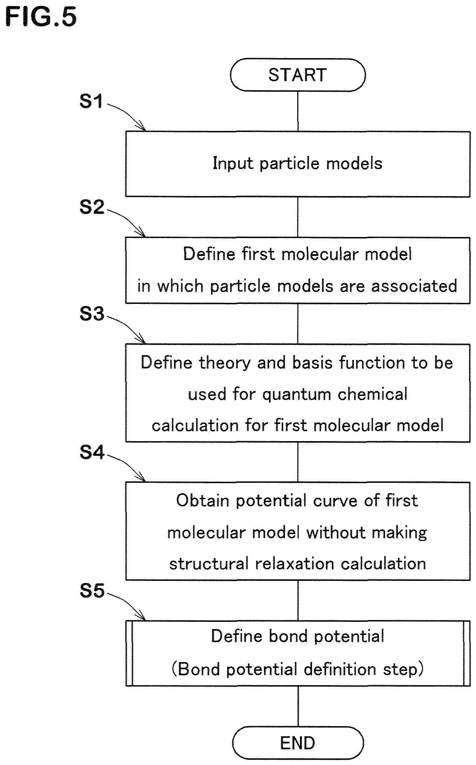

In this embodiment, different bond potentials P1 are respectively defined between the carbon particle models 6C, between the carbon particle model 6C and the hydrogen particle model 6H, between the carbon particle model 6C and the sulfur particle model 6S, and between the sulfur particle model 6S and the hydrogen particle model 6H shown in Fig. 3. Fig. 5 is a flowchart showing an example of the processing procedure of the defining method in this embodiment.

In the defining method in this embodiment, first, at least two particle models 6 modeling at least two atoms are inputted to the computer 1 (step S1). In step S1 in this embodiment, all types of the particle models 6 constituting the molecular chain model 5 shown in Fig. 3 are set. Thereby, in step S1, the carbon particle models 6C modeling carbon atoms, the hydrogen particle models 6H modeling hydrogen atoms, and the sulfur particle models 6S modeling sulfur atoms are each inputted to the computer 1. Details of the particle models 6 are as described above.

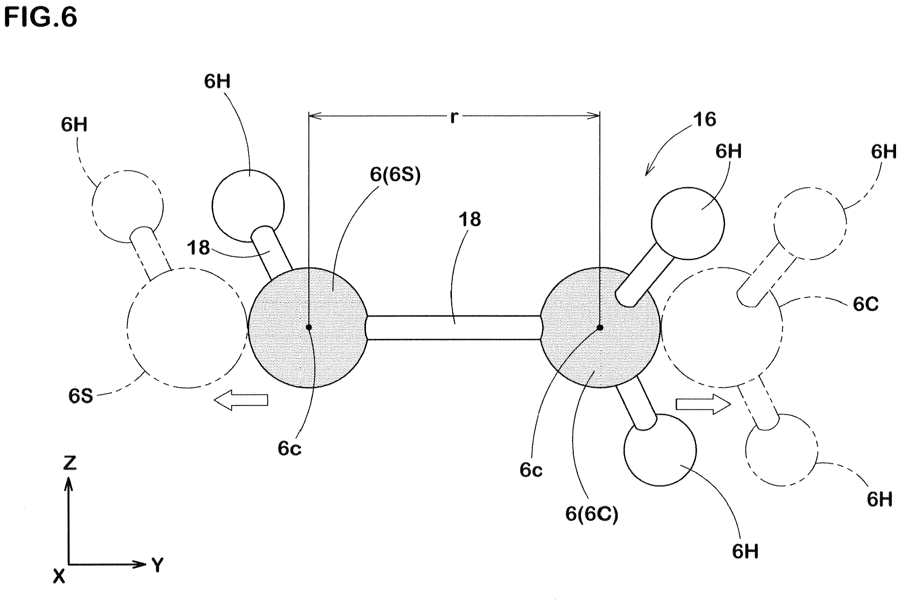

Next, in the defining method in this embodiment, a (step S2). Fig. 6 is a conceptual diagram showing an example of the first molecular model 16.

In step S2 in this embodiment, in the molecular chain model 5 shown in Fig. 3, the first molecular model 16 is defined based on all combinations of a pair of the particle models 6 in which the bond potential P1 is defined. Thereby, in this embodiment, the first molecular model 16 in which the carbon particle models 6C are associated with each other, the first molecular model 16 in which the carbon particle model 6C and the hydrogen particle model 6H are associated with each other, the first molecular model 16 in which the sulfur particle model 6S and the carbon particle model 6C are associated with each other, and the first molecular model 16 in which the sulfur particle model 6S and the hydrogen particle model 6H are associated with each other are set. Fig. 6 shows the first molecular model 16 in which the sulfur particle model 6S and the carbon particle models 6C are associated with each other as a representative example of the first molecular model 16.

As shown in Fig. 6, each of the particle models 6 is bound with the hydrogen particle model 6H via a bond model 18 (potential P2). The number of the bound hydrogen particle models 6H (1, in the case of sulfur atom, for example) is calculated by subtracting the number of bonds of the particle models 6 (1, in the case of sulfur atom, for example) from an atom valence (2 in the case of sulfur atom, for example) of the atom modeled by the particle model 6. Thereby, the first molecular model 16 shown in Fig. 6 is set as methanethiol. The first molecular model 16 configured as such can maintain a stable structure in quantum chemical calculation. The first molecular model 16 is stored in the computer 1.

Next, in the defining method in this embodiment, theory and a basis function to be used for quantum chemical calculation are defined between the particle models 6 of the first molecular model 16 (step S3). As the theory defined in this embodiment, a density functional theory (exchange-correlation functional: B3LYP) is defined, for example. Further, as the basis function, a basis function (6-31G(d)) is defined, for example. The theory and the basis function used for the quantum chemical calculation can be defined by using a quantum chemical calculation program (such as Gaussian 03 or Gaussian 09 available from Gaussian, for example). In step S3, the theory and the basis function used for the quantum chemical calculation are defined for all of the first molecular model 16 described above. These theories and basis functions are stored in the computer 1.

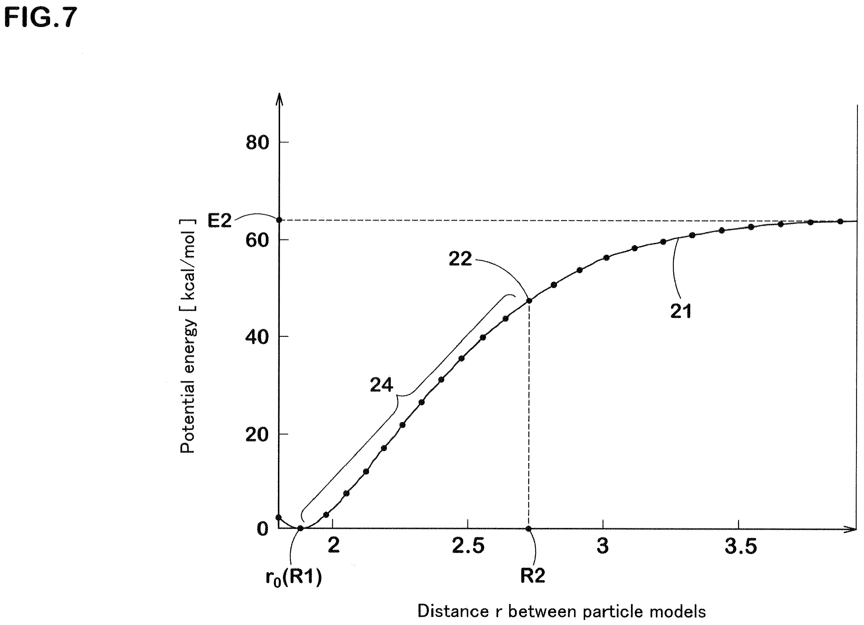

Next, in the defining method in this embodiment, the computer 1 obtains a potential curve showing the relationship between the potential energy of the first molecular model 16 and the distance between at least two particle models 6 (hereinafter may be simply referred to as "potential curve of the first molecular model 16") (step S4). Fig. 7 is a graph showing an example of a potential curve 21 of the first molecular model 16. The graph in Fig. 7 illustrates the potential curve 21 of the first molecular model 16 (shown in Fig. 6) in which the sulfur particle model 6S and the carbon particle model 6C are associated with each other as an example.

As shown in Fig. 7, in step S4, a first distance (equilibrium distance) r0 between at least two particle models 6 (the sulfur particle model 6S and the carbon particle model 6C in this example) in an equilibrium state of the first molecular model 16 shown in Fig. 6 due to balance of the interaction based on the nucleus and the electron is determined by the theory and the basis function described above. In step S4 in this embodiment, quantum chemical calculation is performed in which a distance between the particle models 6 are increased stepwise (by 0.1 angstrom for each unit step, for example) from the state where the particle models 6 are in contact until a distance r between the particle models 6 becomes larger than the equilibrium distance r0 (4.5 angstrom, for example), for example.

Further, in the quantum chemical calculation in this embodiment, the potential energy of the first molecular model 16 is calculated for each unit step based on an unrestricted density functional theory. Thereby, in step S4, as shown in Fig. 7, the potential curve 21 showing the relationship between the potential energy of the first molecular model 16 (shown in Fig. 6) and the distance between at least two particle models 6 (the sulfur particle model 6S and the carbon particle model 6C in this example) is obtained.

In the graph of Fig. 7, the potential curve 21 of the first molecular model 16 can be approximated well to the potential curve 11 of the Morse-type function shown in Fig. 4. Therefore, the repulsive force and the attractive force can be defined according to the distance r by using the potential curve 21 of the first molecular model 16 similarly to the potential curve 11 of the Morse-type function.

The potential curve 21 of the first molecular model 16 has an increase change point 22 similarly to the potential curve 11 (shown in Fig. 4) of the Morse-type function. The increase change point 22 can also be defined in the same way as the increase change point 13 (shown in Fig. 4) of the potential curve 11 of the Morse-type function.

when the distance between the particle models 6 (the sulfur particle model 6S and the carbon particle model 6C in this example) shown in Fig. 6 are increased in step S4, the structure of the first molecular model 16 tends to become unstable. Thereby, in the conventional defining method, structural relaxation calculation for the first molecular model 16 is made each time the distance between the particle models 6 are increased (that is, per unit step). Note that the structural relaxation calculation for the first molecular model 16 can be made by using a function (partial optimization, for example) provided in advance in the quantum chemical calculation program, for example.

However, in the conventional defining method, it is difficult to obtain the potential curve 21 (shown in Fig. 7) close to that in reality, and it is difficult to accurately define the bond potential P1 (shown in Fig. 3). As a result of intensive research, the inventors of the present invention have found that when the structural relaxation calculation for the first molecular model 16 is made each time the distance between the particle models 6 shown in Fig. 6 are increased (that is, every unit step), the two particle models 6, which are to be spaced away from each other, become close to each other by their own attractive force, therefore, there are some cases where it is difficult to calculate potential energy close to that in reality. Further, the inventors have found that it is possible to prevent the two particle models 6, which are to be spaced away from each other, from getting close to each other due to the relaxation by obtaining the potential curve 21 (shown in Fig. 7) of the first molecular model 16 without making the structural relaxation calculation for the first molecular model 16 (that is, without making the structural relaxation calculation for the first molecular model 16 each time the distance between the particle models 6 are increased), therefore, even if the structure of the first molecular model 16 becomes somewhat unstable, it is possible to certainly eliminate unnatural interactions caused by the separated particle models 6 getting close to each other, thereby, it is possible to calculate the potential energy close to reality.

Based on the findings as just described above, in step S4 in this embodiment, the potential curve 21 shown in Fig. 7 of the first molecular model 16 (shown in Fig. 6) is obtained without making the structural relaxation calculation for the first molecular model 16 (that is, without making the structural relaxation calculation for the first molecular model 16 each time the distance between the particle models 6 are increased). Thereby, in step S4, it is possible to prevent the two particle models 6, which are to be spaced away from each other, from approaching each other, therefore, it is possible to calculate the potential energy close to reality as compared with the conventional defining method. Note that the structural relaxation calculation for the first molecular model 16 is not limited to be made in step S4. If it is not made in step 4, it may be made in a step before step S4 of obtaining the potential curve 21 of the first molecular model 16 (that is, in step S2 of defining the first molecular model 16), or in a step after step S4, for example.

In step S4 in this embodiment, the potential curve 21 is calculated for all of the first molecular model 16 described above. Thereby, in step S4, four types of the potential curves are each calculated. The four types of the potential curves are the potential curve (not shown) between the carbon particle models 6C, the potential curve (not shown) between the carbon particle model 6C and the hydrogen particle model 6H, the potential curve 21 (shown in Fig. 7) between the sulfur particle model 6S and the carbon particle model 6C, and the potential curve (not shown) between the sulfur particle model 6S and the hydrogen particle model 6H. Each of the calculated potential curves 21 is stored in the computer 1.

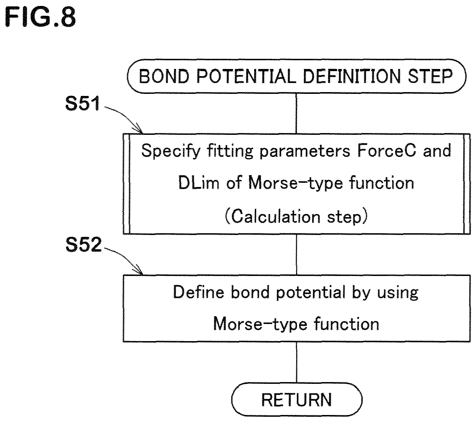

Next, in the defining method in this embodiment, the computer 1 defines the bond potential P1 using the Morse-type function defined by the above formula (1) (bond potential definition step S5). In the bond potential definition step S5, the fitting parameters ForceC and DLim of the Morse-type function are specified so that the Morse-type function approximates the potential curve 21 (shown in Fig. 7) of the first molecular model 16. Fig. 8 is a flowchart showing an example of the processing procedure of the bond potential definition step S5.

In the bond potential definition step S5 in this embodiment, first, the fitting parameters ForceC and DLim of the Morse-type function are specified such that the Morse-type function of the above formula (1) approximates the potential curve 21 of the first molecular model 16 (calculation step S51). In this embodiment, the Morse-type function is approximated with respect to the entire region (the region from the equilibrium distance r0 to 4.5 angstrom, for example) of the potential curve 21 of the first molecular model 16 shown in Fig. 7.

As a method for approximating the Morse-type function to the potential curve 21 of the first molecular model 16, a least squares method can be used, for example. Thereby, in the calculation step S51, the fitting parameters ForceC and DLim of the Morse-type function can be easily specified.

In the calculation step S51 in this embodiment, the Morse-type function is approximated to each of the potential curve (not shown) between the carbon particle models 6C, the potential curve (not shown) between the carbon particle model 6C and the hydrogen particle model 6H, the potential curve 21 (shown in Fig. 7) between the sulfur particle model 6S and the carbon particle model 6C, and the potential curve (not shown) between the sulfur particle model 6S and the hydrogen particle model 6H. Thereby, in the calculation step S51, the fitting parameters ForceC and DLim of the Morse-type function are specified for each of the potential curve (not shown) between the carbon particle models 6C, the potential curve (not shown) between the carbon particle model 6C and the hydrogen particle model 6H, the potential curve 21 (shown in FIG. 7) between the sulfur particle model 6S and the carbon particle model 6C, and the potential curve (not shown) between the sulfur particle model 6S and the hydrogen particle model 6H. The specified fitting parameters ForceC and DLim are stored in the computer 1.

Next, in the bond potential definition step S5 in this embodiment, the bond potential P1 (shown in Fig. 3) is defined by using the Morse-type function in which the fitting parameters ForceC and DLim are specified (step S52).

In step S52, the fitting parameters ForceC and DLim specified by the potential curve (not shown) between the carbon particle models 6C shown in Fig. 3 are assigned to the Morse-type function to define the bond potential P1 between the carbon particle models 6C.

Further, in step S52, the fitting parameters ForceC and DLim specified by the potential curve (not shown) between the carbon particle model 6C and the hydrogen particle model 6H shown in Fig. 3 are assigned to the Morse-type function to define the bond potential P1 between the carbon particle model 6C and the hydrogen particle model 6H.

Furthermore, in step S52, the fitting parameters ForceC and DLim specified by the potential curve 21 (shown in Fig. 7) between the sulfur particle model 6S and the carbon particle model 6C shown in Fig. 6 are assigned to the Morse-type function to define the bond potential P1 between the carbon particle model 6C and the sulfur particle model 6S.

Still further, in step S52, the fitting parameters ForceC and DLim specified by the potential curve (not shown) between the sulfur particle model 6S and the hydrogen particle model 6H shown in Fig. 3 are assigned to the Morse-type function to define the bond potential P1 between the sulfur particle model 6S and the hydrogen particle model 6H. These bond potentials P1 are stored in the computer 1. By defining these bond potentials P1, the molecular chain model 5 shown in Fig. 3 is set.

As described above, the defining method in this embodiment can calculate the potential curve 21 (shown in Fig. 7) of the first molecular model 16 in which the potential energy close to the reality is reflected, respectively. In the defining method in this embodiment, the fitting parameters ForceC and DLim of the Morse-type function are specified so that the Morse-type function (shown in the above formula (1)) approximates the potential curve 21, therefore, it is possible to define the bond potential P1 with high accuracy.

The bond potential P1 configured as such can accurately represent bonds and dissociations between atoms of the molecular chain 3 (shown in Fig. 2). Thereby, when the molecular chain model 5 (shown in Fig. 3) in which the bond potential P1 is defined is used for simulation of a polymer material using the molecular chain model 5, for example, it is possible to analyze cleavage of the molecular chain 3 with high accuracy.

In the defining method in this embodiment, in the calculation step S51, the Morse-type function (shown in the above formula (1)) is approximated with respect to the entire region (the region from the equilibrium distance r0 to 4.5 angstrom, for example) of the potential curve 21 of the first molecular model 16 shown in Fig. 7. However, it is not limited to such embodiment.

As a result of further intensive research, the inventors have found that, in the potential curve 21, influence by the unstable structure of the first molecular model 16 is small in a region 24 between a first distance R1 (that is, a distance between at least two particle models 6 in an equilibrium state of the first molecular model 16 shown in Fig. 6) and a second distance R2 between at least two particle models 6 at the increase change point 22, therefore, the potential energy closer to reality is reflected. Further, the fitting parameter ForceC of the above equation (1) corresponds to curvature of the potential curve 11 of the Morse-type function shown in Fig. 4 in the vicinity of the equilibrium distance RM (i.e., corresponding to the first distance R1 in the potential curve 21 of the first molecular model 16), therefore, it defines the potential energy of the region 24.

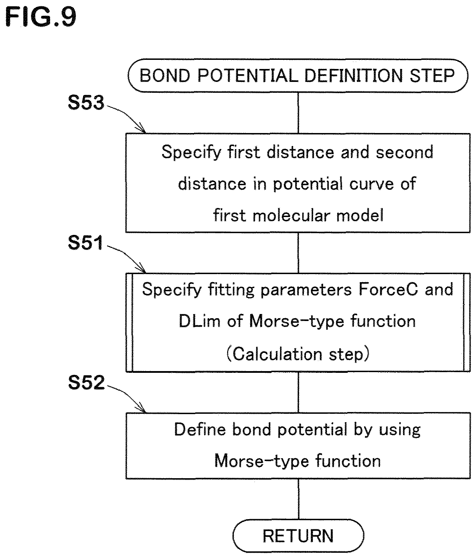

Based on the findings described above, in the calculation step S51 in this embodiment, the fitting parameter ForceC of the Morse-type function is specified such that the Morse-type function is approximated to the region 24 between the first distance R1 and the second distance R2 in the potential curve 21 of the first molecular model 16. Fig. 9 is a flowchart showing an example of the processing procedure of the bond potential definition step S5 according to another embodiment (second embodiment) of the present invention. In the second embodiment, the same components as those of the previous embodiment (the first embodiment) are denoted by the same reference numerals, and the description thereof may be omitted.

In the bond potential definition step S5 in this second embodiment, prior to the calculation step S51, the first distance R1 and the second distance R2 are specified in the potential curve 21 of the first molecular model 16 shown in Fig. 7 (step S53). As described above, the first distance R1 is the distance between two particle models 6 in an equilibrium state of the first molecular model 16 shown in Fig. 6. Further, the second distance R2 is larger than the first distance R1 and is the distance between two particle models 6 at the increase change point 22 where the increase of the potential energy becomes small.

In the step S53, the first distance R1 and the second distance R2 are specified with respect to each of the potential curve (not shown) between the carbon particle models 6C, the potential curve (not shown) between the carbon particle model 6C and the hydrogen particle model 6H, the potential curve 21 (shown in Fig. 7) between the sulfur particle model 6S and the carbon particle models 6C, and the potential curve (not shown) between the sulfur particle model 6S and the hydrogen particle model 6H. These first distances R1 and the second distances R2 are stored in the computer 1.

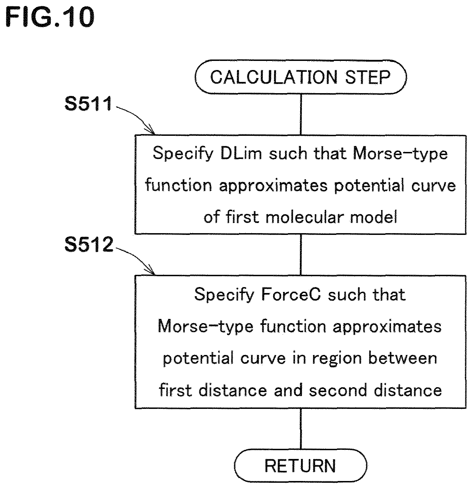

In the bond potential definition step S5 in this second embodiment, the calculation step S51 is performed next. Fig. 10 is a flowchart showing an example of the calculation step S51 according to the second embodiment of the present invention.

In the calculation step S51 in this second embodiment, first, the fitting parameter DLim of the Morse-type function (shown in the above formula (1)) is specified such that the Morse-type function approximates the potential curve 21 (shown in Fig. 7) of the first molecular model 16 (step S511). In the step S511, similarly to the calculation step S51 in the first embodiment, the fitting parameters ForceC and DLim of the Morse-type function are specified such that the Morse-type function is approximated with respect to the entire region (the region from the equilibrium distance r0 to 4.5 angstrom, for example) of the potential curve 21 of the first molecular model 16. of these parameters ForceC and DLim, the fitting parameter DLim is assigned to the Morse-type function used for defining the bond potential P1 in the step S52. Note that the fitting parameter ForceC specified in the step S511 is not assigned to the Morse-type function used for defining the bond potential P1, and the fitting parameter ForceC specified in the next step S512 is assigned to the Morse-type function used for defining the bond potential P1.

In the step S511, the fitting parameter DLim is specified with respect to each of the potential curve (not shown) between the carbon particle models 6C, the potential curve (not shown) between the carbon particle model 6C and the hydrogen particle model 6H, the potential curve 21 (shown in Fig. 7) between the sulfur particle model 6S and the carbon particle models 6C, and the potential curve (not shown) between the sulfur particle model 6S and the hydrogen particle model 6H. The specified fitting parameters DLim are stored in the computer 1.

Next, in the calculation step S51 in this second embodiment, the fitting parameter ForceC of the Morse-type function is specified such that the Morse-type function (shown in the above formula (1)) approximates the region 24 between the first distance R1 and the second distance R2 of the potential curve 21 of the first molecular model 16 shown in Fig. 7 (step S512). In the step S512, the fitting parameter ForceC is specified such that the Morse-type function to which the fitting parameter DLim specified in the step S511 is assigned approximates most to the potential curve 21 in the region 24 between the first distance R1 and the second distance R2. Thereby, with respect to the potential curve 21 of the first molecular model 16, the fitting parameter ForceC reflecting curvature in the vicinity of the first distance R1 is obtained.

The fitting parameter ForceC specified in the step S512 is assigned to the Morse-type function used for defining the bond potential P1 in step S52. Thereby, in the defining method in this second embodiment, it is possible to define the bond potential P1 reflecting the potential energy closer to reality as compared with the first embodiment.

In the step S512, the fitting parameter ForceC is specified with respect to each of the potential curve (not shown) between the carbon particle models 6C, the potential curve (not shown) between the carbon particle model 6C and the hydrogen particle model 6H, the potential curve 21 (shown in Fig. 7) between the sulfur particle model 6S and the carbon particle models 6C, and the potential curve (not shown) between the sulfur particle model 6S and the hydrogen particle model 6H. The specified fitting parameters ForceC are stored in the computer 1.

As described above, in this second embodiment, the fitting parameter ForceC is specified such that the Morse-type function approximates the region 24 between the first distance R1 and the second distance R2 where the potential energy closer to reality is reflected most, therefore, it is possible to define the bond potential P1 (shown in Fig. 3) with high accuracy as compared with the first embodiment. Thereby, when the molecular chain model 5 (shown in Fig. 3) in which the bond potential P1 is defined is used for simulation of a polymer material using the molecular chain model 5, for example, it is useful for analyzing cleavage of the molecular chain with high accuracy. Further, in the step S512, the fitting parameter ForceC is specified by using the Morse-type function to which the fitting parameter DLim is assigned, therefore, it is possible that the Morse-type function effectively approximates the potential curve 21.

In the defining method in the first and the second embodiments, in the calculation step S51, the fitting parameter DLim is specified by making the Morse-type function (shown in the above formula (1)) approximate the potential curve 21 of the first molecular model 16 shown in Fig. 7 in its entire region (the region from the equilibrium distance r0 to 4.5 angstrom, for example). However, it is not limited to such embodiment.

As a result of further intensive research, the inventors have found that, in the step S4, when the distance between the particle models 6 are increased while the structure of the first molecular model 16 shown in Fig. 6 is unstable, bond dissociation energy E2 (shown in Fig. 7) is calculated to be larger than the actual potential energy (including bond dissociation energy). When the Morse-type function is approximated to the potential curve 21 (shown in Fig. 7) of the first molecular model 16 configured as such, the fitting parameter DLim corresponding to the bond dissociation energy is specified to be unnecessarily large, therefore, there is room for further improvement in order to define the bond potential P1 with high accuracy.

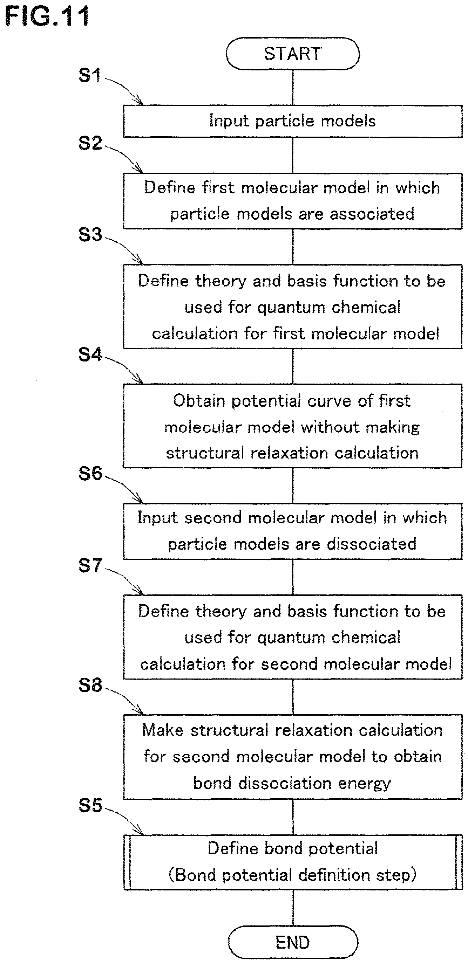

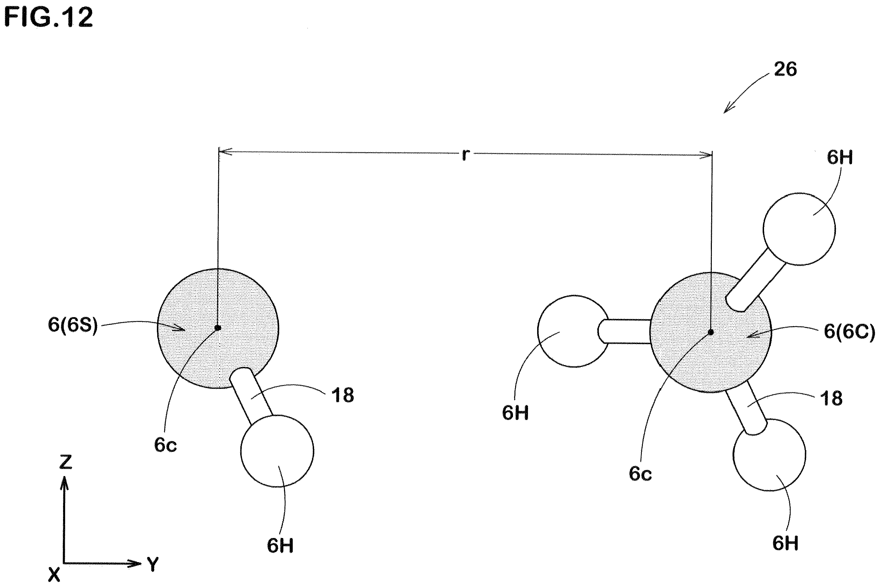

Based on this finding, in the calculation step S51 in this embodiment, the structural relaxation calculation for a second molecular model in which at least two particle models 6 are dissociated is made, and then the fitting parameter DL im of the Morse-type function is specified by using the bond dissociation energy between the at least two particle models 6. Fig. 11 is a flowchart showing an example of the processing procedure of the defining method according to still another embodiment (third embodiment) of the present invention. Fig. 12 is a conceptual diagram showing an example of a second molecular model 26. In the third embodiment, the same reference numerals are given to the same configurations as those of the first and the second embodiments, and the description thereof may be omitted.

In the defining method of the third embodiment, prior to the bond potential definition step S5, the second molecular model 26 in which at least two particle models 6 are dissociated is inputted to the computer 1 (step S6). In the step S6, in the molecular chain model 5 shown in Fig. 3, the second molecular model 26 is defined for each of all combinations of a pair of the particle models 6 in which a bond potential P1 is defined. Thereby, in the third embodiment, four types of the second molecular models are set. The four types of the second molecular models are the second molecular model (not shown) in which the carbon particle models 6C are dissociated, the second molecular model (not shown) in which the carbon particle model 6C and the hydrogen particle model 6H are dissociated, the second molecular model 26 (shown in Fig. 12) in which the sulfur particle model 6S and the carbon particle model 6C are dissociated, and the second molecular model (not shown) in which the sulfur particle model 6S and the hydrogen particle model 6H are dissociated. Fig. 12 shows the second molecular model 26 in which the sulfur particle model 6S and the carbon particle model 6C are dissociated as a representative example of the second molecular model 26.

Unlike the first molecular model 16 shown in Fig. 6, the bond model 18 (potential P2) is not defined between the particle models 6 (between the sulfur particle model 6S and the carbon particle model 6C in this example) of the second molecular model 26. Further, the distance r between the particle models 6 of the second molecular model 26 is set to be larger than the second distance R2 (i.e., the distance between the two particle models 6 at the increase change point 22) shown in Fig. 7. Thereby, it is possible to define the second molecular model 26 in which two particle models 6 are dissociated. The second molecular model 26 is stored in the computer 1.

Next, in the defining method in this third embodiment, the theories and the basis functions to be used for quantum chemical calculation are defined between the particle models 6 of the second molecular model 26 (step S7). The same theories and the basis functions defined between the particle models 6 of the first molecular model 16 shown in Fig. 6 are defined as the theories and the basis functions used for the quantum chemical calculations in this third embodiment. These theories and the basis functions are stored in the computer 1.

Next, in the defining method in this third embodiment, the computer 1 make the structural relaxation calculation for the second molecular model 26 to calculate the bond dissociation energy between at least two particle models 6 (between the sulfur particle model 6S and the carbon particle model 6C in this example) (Step S8). In the step S8, the potential energy of the second molecular model 26 is calculated based on the unrestricted density functional theory. In the second molecular model 26, the two particle models 6 are dissociated. The potential energy of the second molecular model 26 configured as such is calculated as the bond dissociation energy between two particle models 6. Note that the structural relaxation calculation for the second molecular model 26 can be made by the above-described method.

In the step S8, the bond dissociation energy (the potential energy) is obtained in a state where the structure of the second molecular model 26 is stable. Thereby, the bond dissociation energy of the second molecular model 26 is calculated to be smaller than the bond dissociation energy E2 (shown in Fig. 7) which is calculated with respect to the first molecular model 16 having the unstable structure, therefore, the bond dissociation energy of the second molecular model 26 is closer to the bond dissociation energy in reality. The bond dissociation energy of the second molecular model 26 is stored in the computer 1.

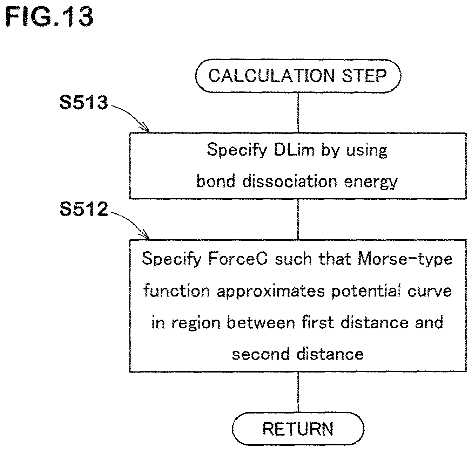

Fig. 13 is a flowchart showing an example of the calculation step S51 according to the third embodiment of the present invention. In the calculation step S51 in this third embodiment, the fitting parameter DLim of the Morse-type function shown in the above formula (1) is specified by using the bond dissociation energy (step S513). The specified fitting parameter DLim is stored in the computer 1.

As described above, in the defining method in this third embodiment, the fitting parameter DLim of the Morse-type function is specified by using the bond dissociation energy of the second molecular model 26 that is closer to the actual bond dissociation energy, therefore, it is possible to define the bond potential P1 (shown in Fig. 3) with high accuracy.

Further, in this third embodiment, similarly to the second embodiment, in the step S512, the fitting parameter ForceC is specified such that the Morse-type function to which the fitting parameter DLim specified in the step S513 is assigned is approximated most to the potential curve 21 in the region 24 between the first distance R1 and the second distance R2. Thereby, in this third embodiment, it is possible to define the bond potential P1 (shown in Fig. 3) with higher accuracy as compared with the first and the second embodiments. when the molecular chain model 5 (shown in Fig. 3) in which the bond potential P1 is defined is used for simulation of a polymer material using the molecular chain model 5, for example, it is useful for analyzing cleavage of the molecular chain with higher accuracy.

While detailed description has been made of an especially preferred embodiment of the present invention, the present invention can be embodied in various forms without being limited to the illustrated embodiments.

**Working Example (Example)**

According to the processing procedure shown in Fig. 5, the bond potential between the atoms was defined by using the Morse-type function (Examples 1 to 3). In Example 1, the potential curve was obtained according to the processing procedure shown in Fig. 8 without making the structural relaxation calculation of the first molecular model. Then, in Example 1, the fitting parameters ForceC and DLim of the Morse-type function were specified by approximating the Morse-type function to the entire region of the potential curve of the first molecular model.

In Example 2, the fitting parameter DL im was specified according to the procedure shown in Figs. 9 and 10, and then the fitting parameter ForceC was specified such that the Morse-type function to which the fitting parameter DLim was assigned approximates the region between the first distance and the second distance of the potential curve of the first molecular model obtained in Example 1.

In Example 3, the fitting parameter DLim of the Morse-type function was specified according to the processing procedure shown in Figs. 9, 11, and 13 by using the bond dissociation energy calculated by using the second molecular model in which the particle models were dissociated. Further, in Example 3, the fitting parameter ForceC was specified such that the Morse-type function to which the fitting parameter DLim specified in Example 3 was assigned approximates the region between the first distance and the second distance of the potential curve of the first molecular model obtained in Example 1.

For Reference, the potential curve was obtained with the structural relaxation calculation for the first molecular model made. In Reference, the fitting parameters ForceC and DLim of the Morse-type function were specified by approximating the Morse-type function to the entire region of the potential curve of the first molecular model. Common specifications are as follows.

Molecular chain model:

polyisoprene (polymerization of 100 isoprene molecules) cross-linked with sulfur (one atom)
Number of particle models (atoms): 1301

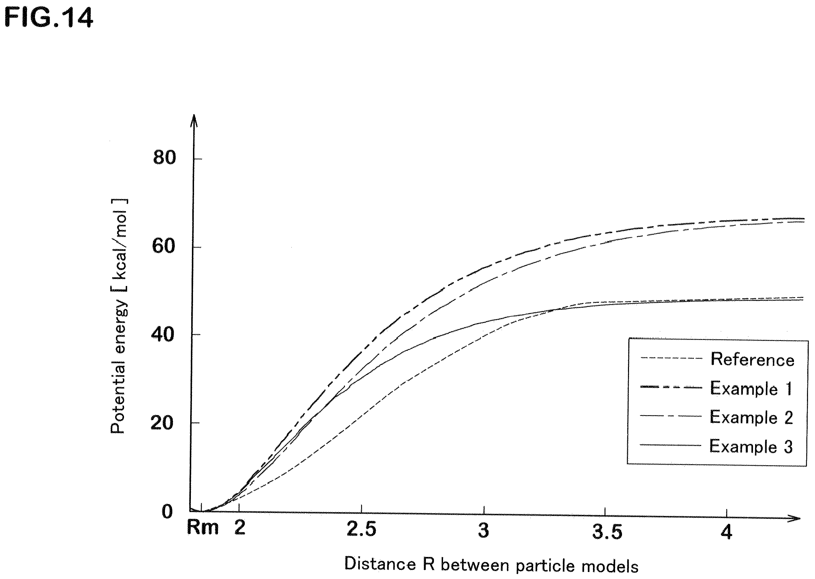

Fig. 14 is a graph showing the potential curves of the Morse-type function of Examples 1 to 3 and Reference. In Examples 1 to 3, as compared with Reference, it was possible to calculate the potential energy close to reality between the equilibrium distance and the increase change point. Thereby, in Examples 1 to 3, it was possible to define the bond potential with high accuracy as compared with Reference.

Examples 2 and 3 showed lower potential energy between the equilibrium distance and the increase change point as compared with Example 1, therefore, it was possible to calculate the potential energy close to reality. Thereby, in Example 2 and Example 3, it was possible to define the bond potential with high accuracy as compared with Example 1.

The potential energy of Example 3 was substantially the same as the potential energy of Example 2 and the behavior of the potential energy at the dissociation limit became the same as Reference, therefore, it was possible to calculate the potential energy closer to reality. Thereby, in Example 3, it was possible to define the bond potential with higher accuracy as compared with Example 2.

**Claims**

1. A method for defining bond potential of polymer material, which is the bond potential between at least two atoms constituting a molecular chain of the polymer material, by using a computer comprising steps of:

   inputting at least two particle models respectively modeling the at least two atoms into the computer;
   defining, in the computer, a first molecular model in which the at least two particle models are associated with each other;
   obtaining, by using the computer, a potential curve indicating relationship between potential energy of the first molecular model and a distance between the at least two particle models without making a structural relaxation calculation for the first molecular model by the computer; and
   defining, by the computer, the bond potential by using Morse-type function defined by the following formula (1):

$$E = DLim \times \left[ exp\left( -\sqrt{\frac{ForceC}{DLim}} \times (R - R_M) \right) - 1 \right]^2 \cdots (1)$$

   wherein E is potential energy, ForceC and DLim are fitting parameters of the Morse-type function, R is a distance between the two particle models, and RM is an equilibrium distance between the two particle models, and
   wherein the step of defining the bond potential includes a calculation step for specifying the fitting parameters ForceC and DLim of the Morse-type function such that the Morse-type function approximates the potential curve.

2. The method for defining bond potential of polymer material according to claim 1 further comprising steps of:

   inputting a second molecular model, in which the at least two particle models are dissociated, into the computer; and
   obtaining, by using the computer, bond dissociation energy between the at least two particle models by making the structural relaxation calculation for the second molecular model: wherein
   in the calculation step, the fitting parameter DLim of the Morse-type function is specified by using the obtained bond dissociation energy.

3. The method for defining bond potential of polymer material according to claim 1 or 2, wherein
the step of defining the bond potential includes a step of specifying, in the potential curve, a first distance between the at least two particle models in an equilibrium state of the first molecular model and a second distance, which is larger than the first distance, between the at least two particle models at an increase change point at which increase of the potential energy becomes small, and
in the calculation step, the fitting parameter ForceC of the Morse-type function is specified such that the Morse-type function approximates the potential curve in a region thereof between the first distance and the second distance.

# FIG.1

FIG.2

## FIG.3

EP 3 418 928 A1

FIG.4

# FIG.5

START

S1

Input particle models

S2

Define first molecular model
in which particle models are associated

S3

Define theory and basis function to be
used for quantum chemical
calculation for first molecular model

S4

Obtain potential curve of first
molecular model without making
structural relaxation calculation

S5

Define bond potential
(Bond potential definition step)

END

# FIG.6

EP 3 418 928 A1

FIG.7

# FIG.8

BOND POTENTIAL DEFINITION STEP

S51

Specify fitting parameters ForceC and

DLim of Morse-type function

(Calculation step)

S52

Define bond potential by using

Morse-type function

RETURN

# FIG.9

BOND POTENTIAL DEFINITION STEP

S53

Specify first distance and second
distance in potential curve of
first molecular model

S51

Specify fitting parameters ForceC and
DLim of Morse-type function
(Calculation step)

S52

Define bond potential by using
Morse-type function

RETURN

# FIG.10

CALCULATION STEP

S511

Specify DLim such that Morse-type function approximates potential curve of first molecular model

S512

Specify ForceC such that Morse-type function approximates potential curve in region between first distance and second distance

RETURN

# FIG.11

START

S1 — Input particle models

S2 — Define first molecular model in which particle models are associated

S3 — Define theory and basis function to be used for quantum chemical calculation for first molecular model

S4 — Obtain potential curve of first molecular model without making structural relaxation calculation

S6 — Input second molecular model in which particle models are dissociated

S7 — Define theory and basis function to be used for quantum chemical calculation for second molecular model

S8 — Make structural relaxation calculation for second molecular model to obtain bond dissociation energy

S5 — Define bond potential (Bond potential definition step)

END

FIG.12

# FIG.13

```
                    ( CALCULATION STEP )
S513                          |
                   ┌──────────────────────────┐
                   │     Specify DLim by using │
                   │   bond dissociation energy │
                   └──────────────────────────┘
S512                          |
                   ┌──────────────────────────┐
                   │ Specify ForceC such that Morse-type │
                   │ function approximates potential curve │
                   │ in region between first distance and │
                   │       second distance      │
                   └──────────────────────────┘
                              |
                        (  RETURN  )
```

**FIG.14**

Europäisches
Patentamt

European
Patent Office

Office européen
des brevets

**EUROPEAN SEARCH REPORT**

Application Number

EP 18 17 6391

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BRAMELD K. ET AL: "Distance Dependent Hydrogen Bond Potentials for Nucleic Acid Base Pairs from ab Initio Quantum Mechanical Calculations (LMP2/cc-pVTZ)", JOURNAL OF PHYSICAL CHEMISTRY PART B, vol. 101, no. 24, 1 June 1997 (1997-06-01), pages 4851-4859, XP055525051, US ISSN: 1520-6106, DOI: 10.1021/jp970199a * the whole document * | 1-3 | INV. G06F19/00 |
| A | NASDALA L. ET AL: "An elastic molecular model for rubber inelasticity", COMPUTATIONAL MATERIALS SCIENCE, ELSEVIER, AMSTERDAM, NL, vol. 106, 16 May 2015 (2015-05-16), pages 83-99, XP029139556, ISSN: 0927-0256, DOI: 10.1016/J.COMMATSCI.2015.04.036 * the whole document * | 1-3 | |
| A | DORUKER P. ET AL: "Simulation of the random scission of C-C bonds in the initial stage of the thermal degradation of polyethylene", COMPUTATIONAL AND THEORETICAL POLYMER SCIENCE, vol. 11, no. 2, 14 September 2000 (2000-09-14), pages 155-166, XP085036716, ISSN: 1089-3156, DOI: 10.1016/S1089-3156(99)00078-1 * abstract * * paragraph [002.] * | 1-3 | TECHNICAL FIELDS SEARCHED (IPC) G06F |

-/--

The present search report has been drawn up for all claims

| Place of search | Date of completion of the search | Examiner |
|---|---|---|
| The Hague | 21 November 2018 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 18 17 6391

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MOLLER J. C. ET AL: "Atomistic prediction of plane stress behavior of glassy thermosets", COMPUTATIONAL MATERIALS SCIENCE, ELSEVIER, AMSTERDAM, NL, vol. 128, 18 December 2016 (2016-12-18), pages 257-277, XP029868064, ISSN: 0927-0256, DOI: 10.1016/J.COMMATSCI.2016.11.019 * the whole document * | 1-3 | |
| A | TANAKA J. ET AL: "New C-F interatomic potential for molecular dynamics simulation of fluorocarbon film formation", JOURNAL OF VACUUM SCIENCE AND TECHNOLOGY: PART A, AVS /AIP, MELVILLE, NY., US, vol. 18, no. 3, 1 May 2000 (2000-05-01), pages 938-945, XP012005074, ISSN: 0734-2101, DOI: 10.1116/1.582279 * abstract * * page 939, right-hand column * | 1-3 | |
| A | REKIK N. ET AL: "Toward accurate prediction of potential energy surfaces and the spectral density of hydrogen bonded systems", PHYSICA B, vol. 436, 13 December 2013 (2013-12-13), pages 164-176, XP028821890, ISSN: 0921-4526, DOI: 10.1016/J.PHYSB.2013.12.003 * the whole document * | 1-3 | |
| A | EP 2 642 417 A2 (SUMITOMO RUBBER IND [JP]) 25 September 2013 (2013-09-25) * the whole document * | 1-3 | |

TECHNICAL FIELDS
SEARCHED (IPC)

The present search report has been drawn up for all claims

| Place of search | Date of completion of the search | Examiner |
|---|---|---|
| The Hague | 21 November 2018 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 18 17 6391

This annex lists the patent family members relating to the patent documents cited in the above-mentioned European search report.
The members are as contained in the European Patent Office EDP file on
The European Patent Office is in no way liable for these particulars which are merely given for the purpose of information.

21-11-2018

| Patent document cited in search report | | Publication date | Patent family member(s) | | Publication date |
|---|---|---|---|---|---|
| EP 2642417 | A2 | 25-09-2013 | CN | 103324825 A | 25-09-2013 |
| | | | EP | 2642417 A2 | 25-09-2013 |
| | | | JP | 5469696 B2 | 16-04-2014 |
| | | | JP | 2013195220 A | 30-09-2013 |
| | | | US | 2013245964 A1 | 19-09-2013 |

For more details about this annex : see Official Journal of the European Patent Office, No. 12/82